## Europäisches Patentamt

(19) **European Patent Office**

**Office européen des brevets**

(11) Numéro de publication: **0 193 555 B1**

(12) **FASCICULE DE BREVET EUROPEEN**

(45) Date de publication du fascicule du brevet: **11.07.90**

(51) Int. Cl.⁵: **C 12 N 15/00**

(21) Numéro de dépôt: **85904261.6**

(22) Date de dépôt: **30.08.85**

(86) Numéro de dépôt international:
**PCT/FR85/00234**

(87) Numéro de publication internationale:
**WO 86/01537 13.03.86 Gazette 86/06**

(54) **APPLICATION DES ANTIBIOTIQUES DE LA FAMILLE DES PHLEOMYCINES A TITRE D'AGENT DE SELECTION DANS LE DOMAINE DU GENIE GENETIQUE.**

(30) Priorité: **31.08.84 FR 8413502**

(43) Date de publication de la demande:
**10.09.86 Bulletin 86/37**

(45) Mention de la délivrance du brevet:
**11.07.90 Bulletin 90/28**

(84) Etats contractants désignés:
**AT BE CH DE FR GB IT LI LU NL SE**

(56) Documents cités:
**EP-A-0 035 831**
**EP-A-0 093 611**

**Journal of Antibiotics, vol. 36, no. 10, octobre 1983, Tokyo (JP); Takeshi Murakami et al.: "Cloning of antibiotic-resistance genes in streptomyces", pages 1305-1311**

**Agric. Biol. Chem., vol. 47, no. 1, janvier 1983; Yasutoshi Takeichi et al.: "Cloning of bacillus subtilis alpha-amylase structural gene in plasmid pUB110", pages 159-161**

(73) Titulaire: **CENTRE NATIONAL DE LA RECHERCHE SCIENTIFIQUE (CNRS)**
**15, Quai Anatole France**
**F-75007 Paris (FR)**

(72) Inventeur: **ARMAU, Elise**
**12, rue J. des Pins**
**F-31300 Toulouse (FR)**
Inventeur: **DROCOURT, Daniel**
**68, rue Bonnat, Bat. E**
**F-31400 Toulouse (FR)**
Inventeur: **ETIENNE, Gilles**
**44, allées Charles de Fitte, Apt 177**
**F-31300 Toulouse (FR)**
Inventeur: **TIRABY, Gérard**
**99, chemin de la Salade Ponsan**
**F-31400 Toulouse (FR)**

(74) Mandataire: **Warcoin, Jacques et al**
**Cabinet Régimbeau 26, avenue Kléber**
**F-75116 Paris (FR)**

Courier Press, Leamington Spa, England.

**EP 0 193 555 B1**

(56) Documents cités:

J. Pharm. Pharmacol. 1980, 32(Suppl.); P. Attfield et al.: "R plasmids mediate protection and sensitivity to bleomycin", page 31P

J. Gen. Appl. Microbiol. 1970, vol. 16, no. 5; Takado Iijima et al.: "Mutability of the phleomycin-resistant mutants of Bacillus Subtilis. I. Isolation of genetically unstable mutants", pages 419-427

## EP 0 193 555 B1

**Description**

La présente invention concerne l'application des phléomycines à titre d'agent de sélection dans le domaine du génie génétique.

Si pour les bactéries on dispose actuellement de marqueurs de sélection fiables et sensibles qui sont constitués par des caractères de résistance à certains antibiotiques tels que l'ampicilline, par contre, pour les cellules eucaryotes les marqueurs de sélection sont, en général, peu sensibles ou nécessitent la mise en oeuvre d'une technologie compliquée pour pouvoir être utilisés.

Il est, en outre, intéressant de disposer d'un marqueur de sélection qui peut s'exprimer aussi bien dans les cellulses procaryotes que dans les cellules eucaryotes puisque souvent les constructions de vecteur dans les cellules eucaryotes sont partiellement effectuées dans des cellulses procaryotes.

La présente invention repose sur la mise en évidence de nouveaux marqueurs de sélection qui confèrent aux cellules une résistance aux antibiotiques de type phléomycine.

La présente invention concerne l'application des antibiotiques de la famille des phléomycines à titre d'agent de sélection de cellules ayant été modifiées artificiellement par incorporation d'un gène phléo$^r$ de résistance à un antibiotique de la famille des phléomycines.

Par "antibiotique de la famille des phléomycines" on etend désigner, outre les phléomycines elles-mêmes, les antibiotiques apparentés tels que les bléomycines, les zorbamycines, la victomycine, les platomycines, les tallysomycines ou les antibiotiques SF 1771, SF 1961 et YA 56, ainsi que les mélanges de ces antibiotiques.

Ces antibiotiques se caractérisent par une structure de base qui les différencie nettement des autres antibiotiques et les essais réalisés dans le cadre de la présente invention ont démontré que la nature exacte de l'antibiotique utilisé pour la sélection ne modifie pas notablement le résultat obtenu pourvu que l'antibiotique soit bien du type phléomycine.

Ces antibiotiques obtenus commercialement (bléomycine) ou préparés à partir des souches productrices ATCC 21890 et 21892 (phléomycines), ATCC 21807 (victomycine), ATCC 21893 (platomycines), ATCC 31158 (tallysomycines), ATCC 31248 (SF 1771) sont relativement toxiques pour les bactéries, les eucaryotes inférieurs comme les levures et les champignons et les eucaryotes supérieurs tant cellules animales que végétales en culture.

Des souches récemment isolées d'Actinomycètes produisent des antibiotiques identiques à ceux mentionnés ci-dessus ou différents. Notamment, la souche V9, Streptoverticillium en cours d'identification taxonomique, synthétise des substances appartenant à la famille des phléomycines. Les courbes d'absorption dans l'ultra-violet des produits purifiés de la souche V9 sont significativement différentes de celles des produits mentionnés ci-dessus.

Les gènes qui confèrent la résistance aux antibiotiques de type phléomycines seront dénomés ci-après de façon générique phléo$^r$.

Il convient dès à présent de signaler que de nombreux gènes phléo$^r$ distincts ont pu être mis en évidence comme cela sera démontré ci-après.

Les gènes phléo$^r$ sont portés par des plasmides naturels isolés de souches bactériennes, par exemple trouvées en milieu hospitalier, ou peuvent être clonés à partir de l'ADN de divers organismes, notamment l'ADN des organismes producteurs des antibiotiques de type phléomycine. Mais ces gènes phléo$^r$ existent également dans des matériaux génétiques largement disponibles puisqu'on a pu mettre en évidence la présence d'un tel gène sur le transposon Tn5 d'*Escherichia coli* ou sur le plasmide pUB110.

Un gène de grande importance pour la présente invention est celui porté par le transposon Tn5 à *E. coli* bien connu des biologistes moléculaires. Ce gène dont la fonction n'a pas été encore décrite est situé dans la partie centrale de Tn5, entre le gène qui entraîne la résistance entre autre à la kanamycine et celui qui entraîne la résistance chez certaines bactéries à la streptomycine.

La localisation exacte du gène en question, désigné par Tn5 phléo$^r$, a été déterminée exactement par dissection de l'ADN de Tn5 avec diverses enzymes de restriction et par détermination de la séquence nucléotidique correspondante.

Le gène Tn5 phléo$^r$ entraîne la résistance à tous les antibiotiques mentionnés ci-dessus, y compris ceux de la souche V9, lorsque celui-ci est rendu fonctionnel après attachement des séquences promotrices et terminales appropriées de l'organisme porteur.

C'est ainsi que le gène Tn5 phléo$^r$ seul est fonctionnel chez les bactéries Gram$^-$ et chez les bactéries Gram$^+$ comme par exemple les bacilles et les streptomyces.

Le gène Tn5 phléo$^r$ entraîne également la résistance à ces antibiotiques dans les levures, à des cellules animales et végétales en culture lorsque celles-ci renferment le gène fonctionnel.

Un deuxième gène digne d'intérêt est porté par le plasmide pUB110 bien connu des généticiens des bacilles. Ce gène est situé sur le plasmide pUB110 en aval du gène qui confère la résistance à la tobramycine. Il est compris dans une région délimitée par le site BglII et qui finit dans le sens contraire des aiguilles d'une montre au site BamHI.

Ce gène désigné 110 phléo$^r$ entraîne la résistance à tous les antibiotiques mentionnés précédemment chez les bacilles porteurs du plasmide pUB110 et également chez les bactéries Gram$^-$ lorsque le gène est mis sous la dépendance des séquences promotrices et terminales appropriées.

Par analogie avec l'exemple du gène Tn5 phléo$^r$, le gène 110 phléo$^r$ devrait également s'exprimer dans

3

les environnements des eucaryotes inférieurs et supérieurs après attachements aux séquences spécifiques à chaque organisme.

Une troisième source de gènes de résistance aux antibiotiques de la famille des phléomycines est fournie par les bactéries productrices de ces antibiotiques.

Un gène désigné SV phléo$^R$ présent sur l'ADN chromosomique de la souche *Streptomyces verticillus* a été cloné sur un plasmide de Streptomyces. Le gène SV phléo$^R$ situé sur le plasmide hybride pUT212 confère la résistance aux antibiotiques de la famille de la phléomycine, à la souche de *Streptomyces violaceoniger* qui héberge le plasmide.

Les cartes physiques des gènes Tn5 phléo$^r$, 110 phléo$^r$ et SV phléo$^r$ déterminées par diverses enzymes de restriction sont dissemblables, ce qui indique une divergence très grande entre les trois gènes.

La mise en oeuvre des antibiotiques de la famille des phléomycines, qui seront parfois appelés ci-après phléomycines par simplification, à titre d'agent de sélection peut être réalisée selon des modalités connues dans le domaine du génie génétique.

Ainsi, lorsque la technique met en oeuvre des vecteurs autoréplicatifs tels que plasmides, phages ou virus, l'agent de sélection est en général utilisé de façon positive ou négative.

Il peut ainsi s'agir d'un procédé de sélection d'une cellule transformée ou transfectée par un vecteur de clonage et/ou d'expression d'une protéine déterminée, caractérisé en ce qu'on introduit dans ce vecteur un gène phléo$^r$ de résistance à un antibiotique de la famille des phléomycines ainsi que les éléments éventuellement nécessaires pour assurer son expression dans les cellules en cause, et en ce que, après transformation ou transfection, on soumet les cellules obtenues à l'action d'un antibiotique de la famille des phléomycines et en ce que l'on sélectionne les cellules résistantes.

Il peut s'agir également d'un procédé de sélection de cellules transformées ou transfectées par un vecteur de clonage et/ou d'expression d'une protéine déterminée ayant intégré un segment d'ADN, caractérisé en ce que l'on introduit ledit segment d'ADN dans le gène phléo$^r$ de résistance aux antibiotiques de la famille des phléomycines porté par ledit vecteur et on soumet les cellules transformées ou transfectées à l'action d'un antibiotique de la famille des phléomycines et on sélectionne les cellules sensibles.

Ce deuxième procédé nécessite évidemment d'avoir conservé un original de la culture pour pouvoir y prélever les colonies sensibles.

Ces procédés sont bien connus dans leur principe, toutefois lorsque, les cellules mises en oeuvre sont des cellules eucaryotes, les rendements de sélection avec les marqueurs de la technique antérieure sont souvent assez faibles et nécessitent l'utilisation de quantités importantes de l'agent de sélection, ce qui dans certains cas n'assure qu'une marge de sélection très faible.

Au contraire, l'utilisation des phléomycines permet une sélection aussi bien chez les procaryotes que chez les eucaryotes avec des quantités faibles d'agent de sélection et avec une très grande sensibilité.

A titre d'exemple, dans le cas de la levure *Saccharomyces cerevisiae,* la souche transformée par un vecteur portant le gène phléo$^r$ résiste à des concentrations de bléomycines considérables, 100 fois supérieures à celles supportées par la souche non transformée.

De façon générale, l'agent de sélection sera utilisé à des concentrations comprises entre 0,1 et 100 µg/ml dans le milieu de culture des cellules modifiées.

Ainsi, on a pu mettre en évidence l'action de ces agents de sélection, aussi bien chez les bactéries Gram$^-$ que Gram$^+$, que chez les levures, les champignons ou les cellules animales ou végétales.

En outre, bien qu'il soit intéressant d'insérer le gène phléo$^r$ sur un vecteur, il est également possible d'intégrer ce gène dans un chromosome cellulaire afin de conférer à la cellule une résistance aux phléomycines.

Bien entendu, la structure des vecteurs évoqués précédemment ne constitue pas en elle-même une caractéristique de la présente invention et ces vecteurs peuvent être préparés par les techniques connues. Il est d'ailleurs évident qu'un certain nombre de vecteurs existant actuellement pourraient être adaptés en insérant le gène phléo$^r$ de façon à pouvoir être manipulés en utilisant les agents de sélection de la présente invention.

Les éléments nécessaires pour assurer l'expression du gène phléo$^r$ dépendent, bien entendu, de la cellule hôte, mais sont connus de l'homme de métier; les exemples ci-après mettront d'ailleurs en évidence certains éléments permettant d'assurer l'expression d'un gène phléo$^r$ suivant la nature de l'hôte.

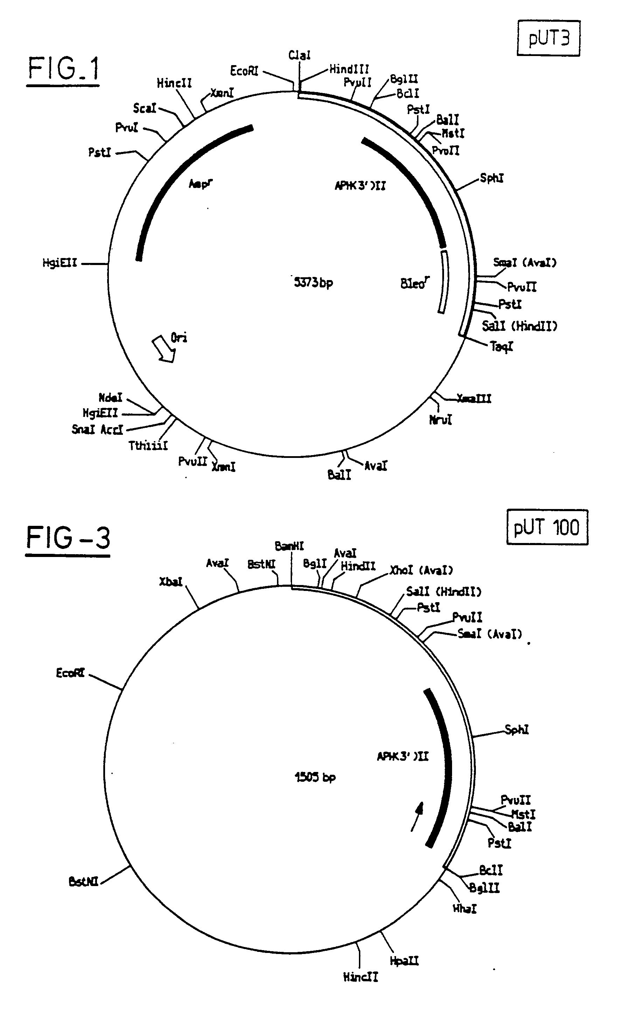

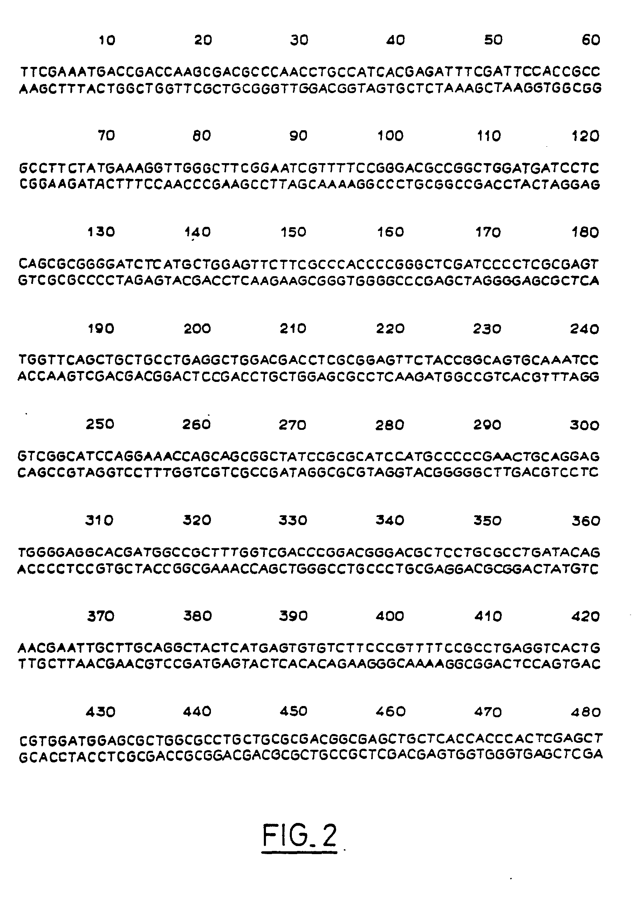

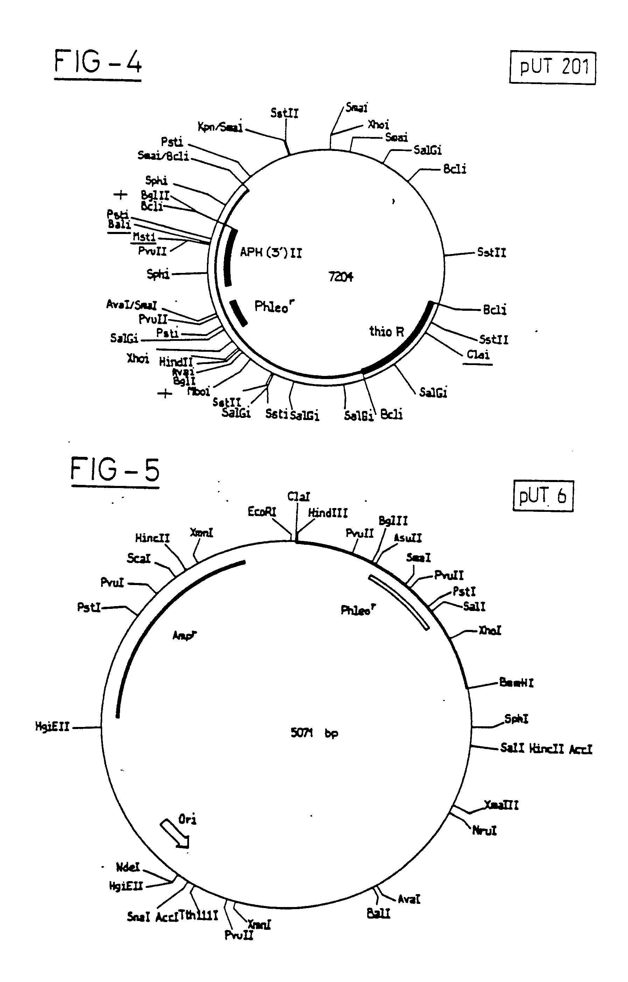

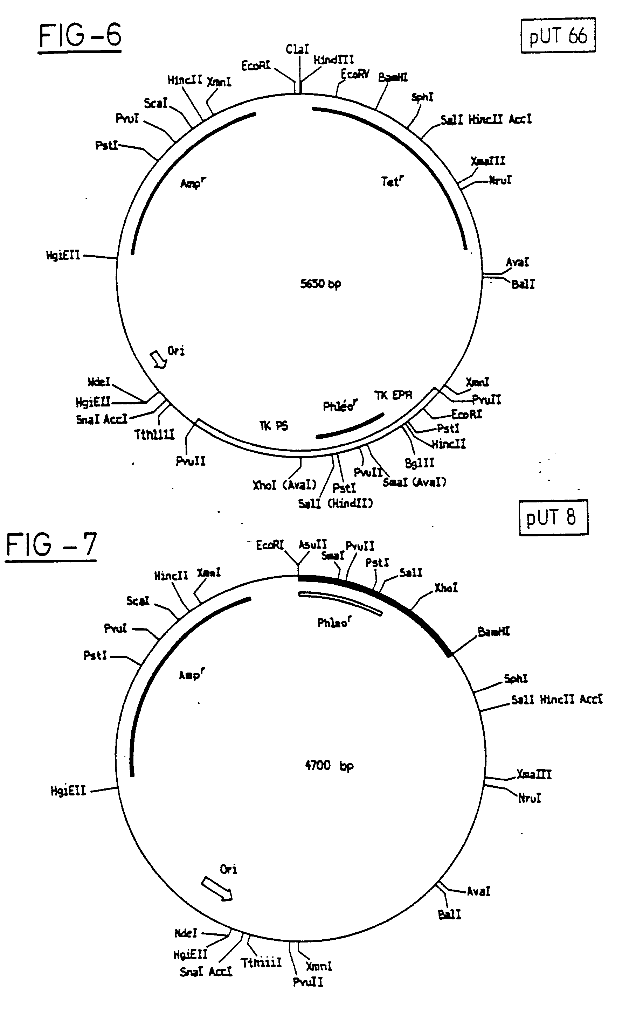

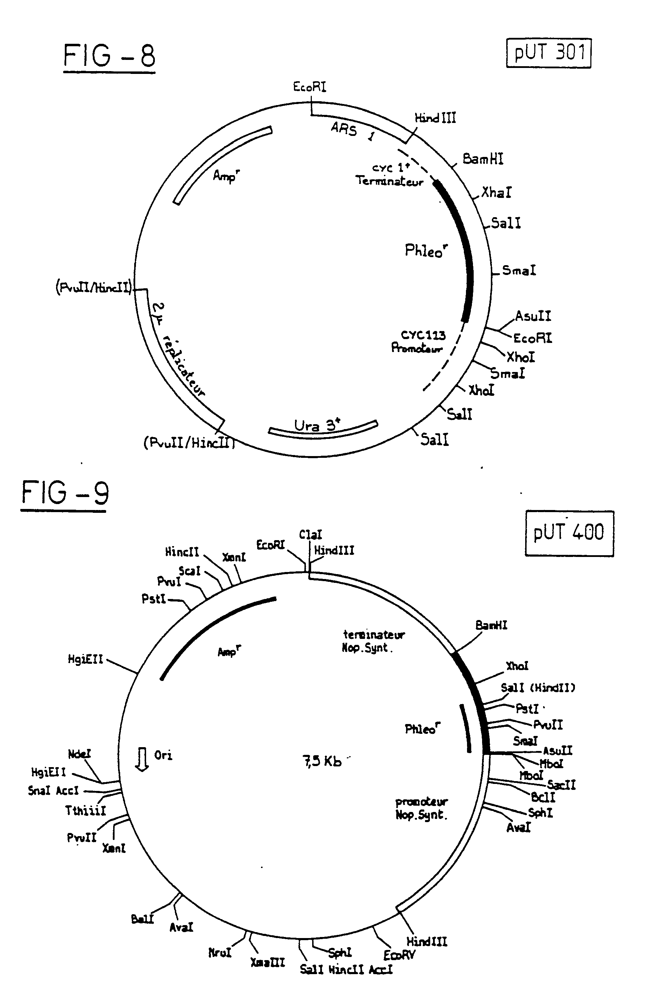

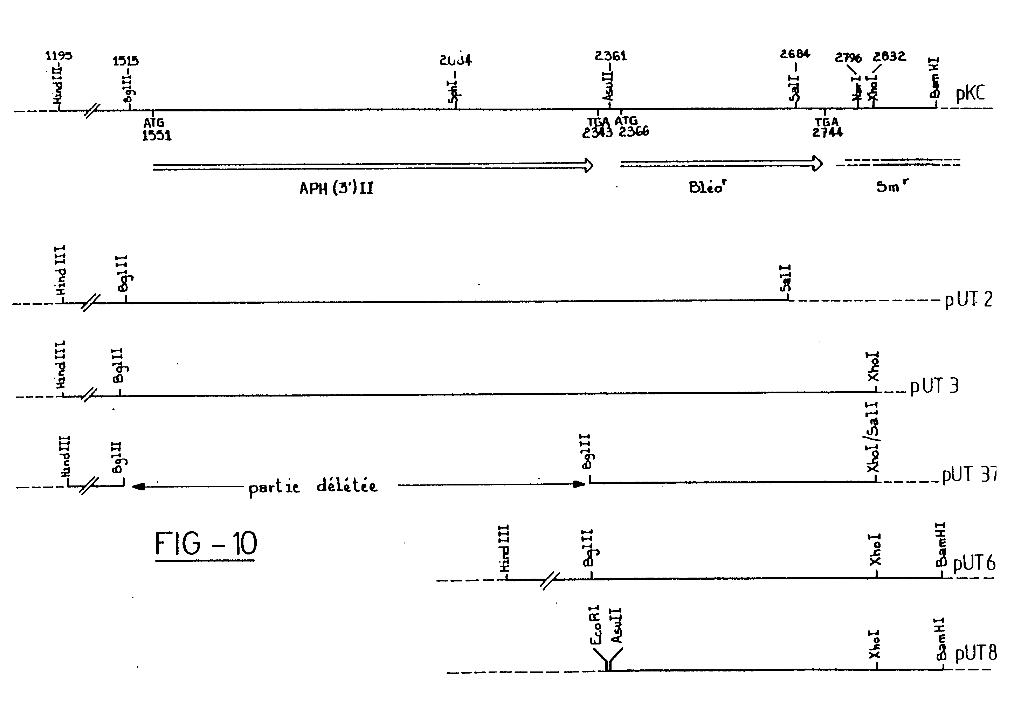

Ces exemples seront décrits en se référant aux figures sur lesquelles:
. la figure 1 est un schéma du plasmide pUT3,
. la figure 2 représente la séquence nucléotidique du gène phléo$^r$ du transposon Tn5,
. la figure 3 est un schéma du plasmide pUT100,
. la figure 4 est un schéma du plasmide pUT201,
. la figure 5 est un schéma du plasmide pUT6,
. la figure 6 est un schéma du plasmide pUT66,
. la figure 7 est un schéma du plasmide pUT8,
. la figure 8 est un schéma du plasmide pUT301,
. la figure 9 est un schéma du plasmide pUT400,
. la figure 10 schématise les différents fragments de restriction de pKC7 mis en oeuvre dans les exemples.

4

Les abréviations utilisées sur ces figures sont considérées comme connues ou seront explicitées dans la description.

Sauf indication contraire, les différents procédés et produisant mentionnés sont mis en oeuvre selon les techniques connues et/ou préconisées par le fabricant.

Exemple I

Des cultures en boîte de Pétri sont préparées à partir de souches d'Actinomycètes productrices d'antibiotiques appartenant au groupe des Bléomycines, obtenues de l'American Type Culture Collection:

| | |
|---|---|
| — Streptomyces verticillus | ATCC 15003 (Bleomycines) |
| — Streptomyces verticillus | ATCC 21678 (Bleomycines) |
| — Streptomyces verticillus | ATCC 21890 (Phléomycines) |
| — Streptomyces flavoridis | ATCC 21892 (Phléomycines) |
| — Streptoalloteichus hindustanus | ATCC 31158 (Tallysomycines) |
| — Streptosporangium violaceochromogenes sub. sp. globophilum | ATCC 21893 (Platomycines) |
| — Streptosporangium violaceochromogenes | ATCC 21807 (Victomycines) |
| — Streptomyces toyocaensis | ATCC 31248 (S.F. 1771) |

La surface des boîtes contenant 30 ml de milieu GAPY (glucose 10; amidon soluble 20; extrait de levure 5; peptone de soja 5; CaCo$_3$ 1; gélose 15 g/l pour 1 l d'eau distillée) est ensemencée à l'anse de platine avec une suspension de spores—mycelia des souches ci-dessus. La production des antibiotiques est évaluée après 15 jours d'incubation à 27°C de la manière suivante: un cylindre de gélose est prélevé de la boîte du milieu de production puis déposé sur un milieu (antibiotique n° 2 Difco+2 g/l glucose, pH 8) ensemencé en profondeur avec des bactéries de la souche HB101 d'*Escherichia coli*. Dans cet exemple deux souches indicatrices sont utilisées: HB101 et HB101 contenant le plasmide pKC7 (RAO et al., Gene 7, 79, 1979). Le plasmide pKC7 isolé à partir de la souche ATCC 37084 a été introduit par transformation dans la souche HB101.

Les résultats de l'antibiogramme exprimés en diamètre d'inhibition (mm) sont présentés dans le tableau 1.

TABLEAU 1

Détermination de la sensibilité de deux souches isogéniques d'*Escherichia coli* aux antibiotiques de la famille des bléomycines

| Souches indicatrices | Souches productrices | | | | | | | |
|---|---|---|---|---|---|---|---|---|
| | 15003 | 21678 | 21890 | 21892 | 31158 | 21893 | 21807 | 31248 |
| HB101 | 31 | 32 | 17 | 18 | 37 | 28 | 16 | 14 |
| HB101 (pKC7) | — | — | — | — | 20 | — | — | — |

Les valeurs indiquées correspondent à la moyenne de 3 cylindres de gélose. L'absence d'auréole d'inhibition est indiqée par un trait.

Il ressort de ce tableau que le plasmide pKC7 protège les cellules qui l'hébergent contre tous les antibiotiques de la famille des bléomycines examinés dans cet exemple. La souche 31158 productrice des tallysomycines synthétise également des antibiotiques du type aminoside dont notamment l'apramycine. L'apramycine testée par la méthode des disques (50 µg) donne une auréole d'inhibition de 30 mm identique pour les deux souches d'*Escherichia coli*. L'inhibition observée pour la souche HB101 (pKC7) par les cylindres de gélose de la souche 31158 est due à la présence de l'apramycine.

Exemple II

Le milieu gélosé correspondant à 20 boîtes de Pétri incubées pendant 10 jours, de milieu de production de la souche de *Streptoalloteichus hindustanus* (ATCC 31158) est broyé 2 fois à l'aide d'un ultraturax en présence chaque fois de 200 ml d'eau puis centrifugé à 5000 rpm. Le surnageant est filtré, puis passé dans une colonne de résine IRC-50 préalablement équilibrée avec de l'acide trifluoroacétique (0,1 mole/l). Les tallysomycines présentes dans le surgnageant s'absorbent sur la résine alors que tous les aminosides ·(nébramycines, tobramycine et apramycine) ne sont pas retenus. Après lavage à l'eau, les tallysomycines sont décrochés avec de l'acide trifluoroacétique 0,01 mole/l. Le volume de l'éluat est réduit de moitié par évaporation à pression réduite et le pH amené à 7. La solution résultante gardée à 4°C est la solution de tallysomycines utilisée dans la suite des essais. Un disque de cellulose inhibée de 20 µl de cette solution donne une auréole d'inhibition de 33 avec HB101 et aucune inhibition avec HB101 (pKC7) dans les conditions décrites dans l'exemple I.

Une solution de bléomycine constitué principalement du composant A5 est préparée de façon identique à partir de 20 boîtes de la souche de *Streptomyces verticillus* (ATCC 21678) supplémenté pour cette souche avec 100 µg/ml de spermidine. Un disque de cellulose inbibé de 20 µl de cette solution de bléomycine A5 donne une auréole d'inhibition de 35 avec HB101 et ne donne pas d'inhibition avec HB101 (pKC7).

Exemple III

La productivité de la souche ATCC 21890 de *Streptomyces verticillus* étant faible en cultures liquides agitées, une amélioration génétique de la souche a été réalisée par deux traitements mutagènes et sélection par la méthode du cylindre de gélose.

$$\text{Souche 21890} \xleftarrow[\substack{\text{lumière} \\ \text{ultra-violette}}]{} \text{A 152-1} \xrightarrow[\substack{\text{nitroso-} \\ \text{guanidine}}]{} \text{B-81 C}$$

La souche mutante B-81 C a été utilisée pour une production des phléomycines en culture liquide: un erlenmeyer de 300 ml contenant 40 ml du milieu inoculum (amidon soluble: 10; extrait de levure: 5; hydrolysat acide de caseine: 5; extrait de viande: 3; glucose: 1; $MgSO_4$: 0,5; $KH_2PO_4$: 0,2; $NaHPO_4$: 0,2; $CaCO_3$: 0,5; g par litre, pH 7 avant stérilisation) est ensemencé avec 0,5 ml d'une suspension de spores-mycelium préparés à partir d'une boîte de milieu GAPY de la souche mutante B-81 C de Streptomyces verticillus. Après 40 heures d'incubation à 27°C sur un agitateur rotatif (220 rpm) 5 ml de la culture inoculum sont utilisés pour inoculer un Erlenmeyer de 2 litres contenant 200 ml du milieu de production (amidon soluble: 20; glucose: 10; Pharmamedia: 10; extrait soluble de maïs: 10; sulfate d'ammonium: 3; $CaCO_3$: 4 g par litre, pH 6,5 avant stérilisation). Après 7 jours de fermentation à 27°C sur agitateur rotatif (250 rpm/min) la culture correspondant à 5 erlenmeyers est filtrée. Le filtrat (900 ml) est passé dans une colonne (40 cm×2 cm) de résine IRC-50 préalablement équilibrée avec de l'acide trifluoroacétique (0,1 mole/l). Après lavage avec 1 litre d'eau distillée les phléomycines sont élués avec 300 ml d'acide trifluoro-acétique 0,02 mole/l. L'éluat neutralisé est passé directement sur une colonne (30×2 cm) de résine XAD-2 préalablement équilibrée avec de l'eau. Après lavage de la colonne avec 250 ml d'eau distillée, les phléomycines sont élués avec 100 ml de méthanol à 80% dans HCl 0,1N. L'éluat évaporé à sec donne 210 mg d'une poudre bleue constituée principalement de phléomycines complexés avec $Cu^{++}$. La poudre obtenue par ce protocole est exempte de l'antibiotique actif sur les bactéries Gram$^+$ distinct des bléomycines et de l'antifongique polyénique produits conjointement par la souche 21890 et ses mutants. L'activité de la poudre phléomycine est comparée à celle de la bléomycine commercialisée par les Laboratoires Roger Bellon (France) et de la pépléomycine en expérimentation clinique (Laboratoires Roger Bellon). Le tableau 2 indique les résultats de l'antibiogramme de disques chargés avec 20 µl de solutions à 1 mg/ml de chacun des antibiotiques. Les solutions de couleur bleue de bléomycine et de pépléomycine complexés par $Cu^{++}$ sont préparées par addition de chlorure cuivrique aux solutions blanches de bléomycine et de pépléomycine. La tétracycline est mise comme contrôle à la même concentration. Dans cet exemple deux souches de *Bacillus subtilis* sont ajoutées. La souche 168 est bien connue des généticiens des bacilles, de même que le plasmide pUB110 qui a été introduit par transformation dans la souche 168.

EP 0 193 555 B1

TABLEAU 2

Antibiogramme de deux souches isogéniques d'*Escherichia coli* et de
deux souches isogéniques de *Bacillus subtilis* aux antibiotiques
de la famille des bléomycines
(milieu antibiotique DIFCO n° 2+2 g/l glucose, pH 8, disques de 10 mm)

| | Souches indicatrices | Phléomycine Cu$^{++}$ | Bléomycine | Bléomycine Cu$^{++}$ | Pépléomycine | Pépléomycine Cu$^{++}$ | Tétracycline |
|---|---|---|---|---|---|---|---|
| *Escherichia coli* | HB101 | 31 | 25 | 24 | 26 | 27 | 21 |
| | HB101 (pKC7) | — | — | — | — | — | 22 |
| *Bacillus subtilis* | 168 | 34 | 26 | 26 | 30 | 29 | 28 |
| | 168 (puB110) | — | — | — | — | — | 28 |

# EP 0 193 555 B1

Il ressort très clairement de ces résultats que le plasmide pKC7 d'une part et le plasmide pUB110 d'autre part protègent leurs cellules hôtes contre l'action toxique des différents composants des phléomycines et des bléomycines. La nature de l'amine terminale du noyau bléomycine ou du noyau phléomycine ainsi que la forme complexée ou pas avec le $Cu^{++}$ n'influent pas sur cette protection.

## Exemple IV

Le plasmide pKC7 qui entraine la résistance à la kanamycine et aux bléomycine du fait du fragment HindIII-BamHI du transposon Tn5 est délété du petit fragment SalI-SalI après digestion complète et ligation. Les cellules HB101 renfermant le plasmide résultant pUT2 sont résistantes à la kanamycine mais sensibles aux bléomycines. Le plasmide pUT3 (figure 1) qui résulte de la ligation du grand fragment HindIII-SalI de pUT2 (digestion de pUT2 par SalI et HindIII et électroélution du gel d'agarose) et du petit fragment HindIII-XhoI de pKC7 (coupure de pKC7 par HindIII et XhoI puis électroélution) conduit à la résistance à la kanamycine et aux bléomycines.

La transformation de HB101 par l'ADN du plasmide pUT3 coupé par SpHI puis digéré par Bal31 pendant des temps variables (5 min., 10 min. 20 min.) suivi de l'addition de linkers BglII phosphorylés et coupure BglII puis ligation a donné des clones transformants ampicilline résistants tous sensibles à la kanamycine et pour moitié environ résistants aux bléomycines au temps 5 min (aux temps 10 et 20 min tous les clones étaient sensibles aux bléomycines). Le plasmide pUT37 est extrait de l'un des clones sensible à la kanamycine et résistant à une forte concentration de bléomycine commerciale.

Ces résultats montrent très clairement que le gène de résistance aux bléomycines du transposon Tn5 est distinct de celui qui code pour une aminoside 3' phosphotransférase de type II responsable de la résistance à la kanamycine et à la généticine. La séquence du gène de structure désigné indifféremment Tn5 Phleo' ou Tn5 Bleo' est donnée dans la figure 2.

## Exemple V

Le petit fragment BglII-BamHI de pKC7 obtenu par double digestion et électroélution d'un gel d'agarose a été ligaturé avec le gros fragment BglII-BamHI du plasmide pUB110 de *Bacillus subtilis*. Après transformation des protoplastes de la souche 168 de *Bacillus subtilis* les clones obtenus sur le milieu de régénération DM3 supplémenté avec 100 µg/ml de kanamycine ont été vérifiés pour leur sensibilité à la tobramycine (10 µg/ml, milieu DIFCO antibiotique n° 2, pH 8) et leur plasmide extrait. La taille de la majorité des plasmides présentait la valeur attendue pour la construction recherchée avec le fragment inséré dans un seul sens, celui où les deux sites BglII et BamHI sont restaurés (plasmide pUT100—figure 3). Les cellules de *Bacillus subtilis* renfermant le plasmide pUT100 sont résistantes à la bléomycine commerciale et aux mêmes aminosides que ceux inactivés par l'APH3'II du transposon Tn5. Ainsi le fragment BglII-BamHI de la partie du Tn5 porté par pKC7, dépourvu de région promotrice exprime les deux types de résistance dans la construction décrite pour ce exemple, chez *Bacillus subtilis* bactérie Gram⁺.

De même, le caractère de résistance à la bléomycine commerciale peut être obtenu chez les Streptomyces, bactéries filamenteuses de grande importance dans l'industrie des fermentations.

Le petit fragment BglII-BamHI de pKC7 a été inséré au site BglII du plasmide pIJ702 (KATZ et al., Journal of General Microbiology, 1983, 129, 2703). Le plasmide hybride résultant pUT201 (figure 4) caractérisé par doubles digestions entraîne la résistance à la kanamycine et à la bléomycine commerciale lorsque celui-ci est présent chez *Streptomyces lividans* et *Streptomyces violaceoniger.*

## Exemple VI

Cet exemple est donné comme illustration de l'intérêt des bléomycines pour sélectionner des clones transformés dans le cas d'expérience de transfert génétique chez les eucaryotes supérieurs.

Le petit fragment HindIII-SalI de pUT37 est ligaturé avec le plus gros fragment HindIII-SalI de pKC7 pour donner le plasmide pUT6 (figure 5). Ce plasmide pUT6 est coupé avec XhoI, les bouts collants rendus nets par action de la polymérase Klenow et après coupure par BglII, le petit fragment BglII-XhoI bout net est électroélué d'un gel d'agarose. Le fragment est ligaturé avec le gros fragment BglII-SmaI du plasmide pAG60 (COLBERE-GARAPIN et al., J. Mol. Biol. (1981) 150, I) pour donner le plasmide pUT66 (figure 6).

L'ADN du plasmide pUT66 est utilisé pour la transfection de la lignée L de cellules de souris thymidine kinase déficiente (TK⁻) selon la méthode décrite par COLBERE-GARAPIN et al., Proc. Nat. Acad. Sci. USA (1979), 76, 3755). La supplémentation de 75 µg/ml de bléomycine commerciale au milieu de croissance entraîne la léthalité de toutes les cellules en 10 jours d'incubation. L'addition de 100 µg/ml de bléomycine commerciale deux jours après la transfection (2 µg d'ADN de pUT6 pour $10^6$ cellules par boîte) suivie de 3 semaines d'incubation permet la sélection de 50 clones en moyenne par boîte, transformés pour le caractère de résistance à la bléomycine. Le nombre de clones transformés est essentiellement le même lorsque la sélection est pratiquée aux deux concentrations testées: 100 µg et 200 µg/ml de bléomycine commerciale. Il est important de signaler que lorsque le plasmide résultant du remplacement du fragment BglII-XhoI de pUT66 par le fragment correspondant de pKC7 est utilisé pour transférer les cellules de souris, seuls les transformants pour la résistance à la généticine (G418) mais non aux bléomycines peuvent être sélectionnés. Ce résultat est en accord avec le fait que chez les eucaryotes, contrairement aux procaryotes, la traduction d'un ARN messager polycistronique n'est pas réinitiée après un codon de terminaison de traduction.

8

Exemple VII

Cet exemple a pour but de montrer l'intérêt des bléomycines pour la sélection des clones transformés chez les eucaryotes inférieurs et particulièrement *Saccharomyces cerevisiae*.

Le gros fragment EcoRI-AsuII de pKC7 obtenu par double digestion EcoRI et AsuII, électroélué d'un gel d'agarose est traité par la polymérase Klenow pour transformer les bouts collants en bouts nets, suivi d'une ligation. La transformation de HB101 donne des clones résistants à l'ampicilline, faiblement résistants à la bléomycine commerciale. La concentration minimale inhibitrice de ces clones porteurs du plasmide pUT8 (figure 7) est de 0,5 µg/ml par comparaison à 0,1 µg/ml pour la souche HB101 et >100 µg/ml pour HB101 (pKC7) dans le milieu antibiotique n° 2+2 g de glucose, pH 8. La faible résistance à la bléomycine entrainée par pUT8 est due à la perte complète de la séquence promotrice (Pribnov box et site de fixation des ribosomes) dans la région délétée. Les sites EcoRI et AsuII sont restaurés dans cette construction. L'ADN du plasmide pEX-2 (GRITZ et DAVIES, Gene (1983) 25: 179) est linéarisé par digestion complète avec BamHI. Après digestion partielle avec EcoRI, le fragment dont la taille correspond au plasmide linéaire est électroélué d'un gel d'agarose et ligaturé avec le petit fragment EcoRI-BamHI de pUT8. Après transformation de HB101, un des clones sélectionnés sur ampicilline et résistant à 2 µg/ml de bléomycine commerciale est retenu comme source du plasmide pUT301 (figure 8).

La souche OL 1 (α, leu 2—3, leu 2—112, his 3—11, his 3—15, ura 3—251, ura 3—373, BOY MARLOTTE et JACQUET, Gene (1982) 20: 433) de *Saccharomyces cerevisiae* est transformée par l'ADN de pUT301 selon la méthode décrite par KLEBE et al. Gene (1983) 25: 333) modifiée de la façon suivante: les cellules traitées par le polyéthylène glycol pendant une heure à 30 minutes sont lavées une fois et resuspendues dans 5 ml de milieu 60% YPD—40% NB. Après 6 heures d'incubation à 30° les cellules sont centrifugées et le culot repris par 1 ml de milieu NB. Les cellules après dilution sont étalées au rateau soit dans le milieu YNB supplémenté avec 100 µg/ml d'histidine et 100 µg/ml de leucine, soit dans le milieu DIFCO n° 2+5 g/l glucose, pH 7,5, additionné de 50 µg/ml de bléomycine commerciale. Après 3 jours d'incubation à 30°C le nombre de clones transformés pour le caractère de résistance à la bléomycine est sensiblement le même que le nombre de clones transformés pour le caractère ura$^+$ ($10^3$ transformés par µg d'ADN). Le contrôle des clones a montré d'une part que tous les clones ura$^+$ étaient résistants à au moins 150 µg/ml de bléomycine commerciale et d'autre part que les clones sélectionnés pour la résistance à la bléomycine étaient tous ura$^+$.

Exemple VIII

Cet exemple est destiné à montrer que le gène de résistance à la bléomycine de pUB100 est différent de celui qui code pour la synthèse de l'aminoglycoside transférase AAD 4' responsable de la résistance à la kanamycin et à la tobramycine.

L'insertion de fragments Sau3A de l'ADN du bactériophage 4 au site BglII de pUB110 donne des clones après transformation des protoplastes de *Bacillus subtilis* résistants à la bléomycine mais sensibles à la kanamycine et à la tobramycine (plasmides de la série pUT102). Inversement, la délétion des deux plus petits fragments HpAII de pUB110 (digestion partielle de l'ADN de pUB110 avec HpaII et électroélution à partir d'un gel d'agarose d'une bande d'environ 3,7 kilobases refermée sur elle-même par ligation) conduit au plasmide pUT101 qui exprime chez *Bacillus subtilis* la résistance à la kanamycine et non à la bléomycine.

Exemple IX

Les exemples précédents ont montré que deux gènes distincts de résistance aux antibiotiques de la famille des bléomycines se trouvaient portés l'un par le transposon Tn5 d'*Escherichia coli* et d'autre part par le plasmide pUB110 de Staphylococcus et *Bacillus subtilis*. La source de gène de résistance aux bléomycines ne se limite pas à ces deux cas. Des plasmides isolés à partir de souches bactériennes isolées en milieu hospitalier peuvent posséder l'information pour ce type de résistance. Par exemple, le plasmide JR66 (DATTA et HEDGES, J. Gen. Microbiol. (1973) 77: 11) entraîne la résistance aux bléomycine ainsi qu'à de nombreux aminosides pour les cellules d'*Escherichia coli* qui l'héberge.

Une autre origine de gènes pouvant entraîner un phénotype de résistance aux bléomycines et fournie par l'ADN chromosomique des Actinomycètes producteurs de ces antibiotiques. Un tel gène a été cloné à partir de l'ADN chromosomique de la souche de *Streptomyces verticillius* (SV) ATCC 21890 productrice des phléomycines et résistante à au moins 200 µg/ml des phléomycines ou de bléomycine commerciale. L'ADN de la souche 21890 préparé selon la méthode de MARMUR (J. Mol. Biol. 1961, 3: 208) est purifié sur DEAE-cellulose puis digéré partiellement avec MboI pour obtenir des fragments compris entre 2 et 6 kilobases. Le produit de la ligation de ces fragments avec l'ADN de pIJ702 ouvert par BglII est utilisé pour transformer les protoplastes de *Streptomyces violaceoniger* selon la méthode décrite par THOMPSON et al. J. Bacteriol. (1982) 151: 668. L'arrêt de la mise en contact des protoplastes avec l'ADN en présence du polyéthylène glycol est réalisé par dilution au 1/10 dans un milieu hypertonique et étalement à cette dilution sur des boîtes contenant le milieu de régénération R2 non sélectif. Après une semaine d'incubation, la surface des boîtes est grattée et la suspension spores-mycélium est étalée sur des boîtes de milieu GAPY additionné de 20 µg/ml de phléomycine et 50 µg/ml de Nosiheptide (antibiotique voisin du thiostrepton). Au cours de trois expériences distinctes, un seul clone stable, capable de se développer en présence de 20 µg de phléomycine, a pu être isolé. Le plasmide pUT212 extrait de ce clone est capable de transformer

# EP 0 193 555 B1

*Streptomyces violaceoniger* pour le caractère de résistance aux phléomycines mais aussi aux autres antibiotiques testés: bléomycine commerciale, pépléomycine, bléomycine A5 et tallysomycines.

Exemple X

Le transposon Tn5 fonctionnel chez un grand nombre de bactéries Gram⁻ est très utilisé en biologie moléculaire pour des expériences de mutagénèse par insertion. L'insertion du transposon Tn5 dans un ADN chromosomique, plasmidique ou phagique, est suivie grâce au caractère de résistance à la kanamycine. La résistance à la bléomycine peut tout aussi bien être exploitée pour la sélection. Ceci a été vérifié en particulier avec des mutants différents d'insertion du Tn5 dans l'ADN chromosomique chez *Rhizobium meliloti* souche 41. Tous les mutants examinés se sont révélés résistants à la fois à la kanamycine et aux bléomycines. Il faut signaler que le plasmide pRme:Tn5 utilisé pour ces expériences entraîne une résistance supérieure à 300 µg/ml de bléomycine commerciale pour les cellules de la souche 41 de *Rhizobium meliloti* alors qu'en absence du plasmide les cellules sont tuées à une concentration égale ou inférieure à 5 µg/ml dans le milieu TY à pH 8.

Dans le domaine de la biologie moléculaire végétale les marqueurs de résistance aux bléomycines peuvent s'avérer d'une grande utilité. C'est ainsi que les cellules dérivées de protoplastes de tabac sont tuées à 1 µg/ml de bléomycine commerciale dans le milieu C (MULLER et al., Physiol. Plant. (1983) 57: 37). Le gène de structure Tn5 Phléo$^r$ (figure 2) mis sous la dépendance de séquences promotrices et terminales spécifique aux cellules végétales devrait pouvoir être exploité de façon bénéfique pour le transfert de gène chez les plantes. Un plasmide porteur du gène Tn5 Phléo$^r$ sous le contrôle des séquences 5' et 3' non traduites de la nopaline synthétase a été construit. L'ADN du plasmide pLGV23 Neo (HERRERA-ESTRELLA et al., EMBO J. (1983) 6: 987) extrait de la souche d'Escherichia coli GM 99 (dam⁻) est linéarisé par BamHI et digéré partiellement par BclI. Le fragment de taille approximative 6,7 kilobases est électroélué puis ligaturé avec le petit fragment BglII-BamHI de pUT6 pour donner le plasmide pUT400 (figure 9). Les cellules de HB101 porteuses de pUT400 sont faiblement résistantes aux bléomycines (c.m.i.: 5 µg/ml de bléomycine commerciale). Le plasmide pUT400 devrait s'avérer particulièrement intéressant pour le clonage de gènes chez les vététaux soit par l'intermédiaire d'Agrobacterium tumefaciens ou Agrobacterium rhizogenes et de leur plasmide respectif Ti et Ri soit par transfection directe de protoplastes de cellules végétales après encapsidation de l'ADN dans des liposomes (CABOCHE et al., Colloque Biologie Moléculaire Végétale, ORSAY (1984) Poster p. 62).

A titre de référence pour l'identification des antibiotiques de la famille des phléomycines, on pourra consulter "Handbook of Antibiotic Compounds" ed. Berdy CRC press, 1980, volume 4.

La pépléomycine est notmment décrite dans l'article de TAKAHASHY et al., J. Antibiotics 1979, 27, 36.

## Revendications

1. Application des antibiotiques de la famille des phléomycines à titre d'agent de sélection:
— de cellules eucaryotes ayant été modifiées artificiellement par incorporation d'un gène phléo$^r$ de résistance à un antibiotique de la famille des phléomycines, ou
— de cellules procaryotes ayant été modifiées artificiellement par incorporation d'un gène phléo$^r$ constitué par tout ou partie de Tn5 ou de pUB110 ou d'un gène phléo$^r$ provenant d'un chromosome d'un organisme produisant un antibiotique de la famille des phléomycines.

2. Application selon la revendication 1, caractérisée en ce que les cellules en cause ont été transformées ou transfectées par un vecteur de clonage ou d'expression portant le gène phléo$^r$ qui s'exprime dans lesdites cellules.

3. Application selon la revendication 1, caractérisée en ce que le gène phléo$^r$ a été intégré dans un chromosome desdites cellules.

4. Application selon l'une des revendications 1 à 3, caractérisée en ce que le gène phléo$^r$ est constitué par tout ou partie de Tn5 ou de pUB110.

5. Application selon l'une des revendications 1 à 3, caractérisée en ce que le gène phléo$^r$ provient d'un chromosome d'un organisme produisant un antibiotique de la famille des phléomycines.

6. Application selon l'une des revendications 1 à 5, caractérisée en ce que les cellules sont des cellules procaryotes.

7. Application selon l'une des revendications 1 à 5, caractérisé en ce que les cellules sont des cellules eucaryotes.

8. Application selon l'une des revendications 1 à 7, caractérisée en ce qu'on utilise l'agent de sélection à une concentration comprise entre 0,1 µg/ml et 100 µg/ml dans le milieu de culture des cellules modifiées artificiellement.

9. Procédé de sélection d'une cellule eucaryote transformée ou transfectée par un vecteur de clonage et/ou d'expression
caractérisé en ce qu'on introduit dans ce vecteur un gène phléo$^r$ de résistance à un antibiotique de la famille des phléomycines ainsi que les éléments éventuellement nécessaires pour assurer son expression dans les cellules en cause, et en ce que, après transformation ou transfection, on soumet les cellules obtenues à l'action d'un antibiotique de la famille des phléomycines et en ce que l'on sélectionne les cellules résistantes.

10. Procédé de sélection d'une cellule eucaryote transformée ou transfectée par un vecteur de clonage et/ou d'expression

ayant intégré un segment d'ADN, caractérisé en ce qu'on introduit ledit segment d'ADN dans le gène phléo$^r$ de résistance aux antibiotiques de la famille des phléomycines porté par ledit vecteur et on soumet les cellules transformées ou transfectées à l'action d'un antibiotique de la famille des phléomycines et on sélectionne les cellules sensibles.

11. Procédé de sélection d'une cellule procaryote transformée ou transfectée par un vecteur de clonage et/ou d'expression

caractérisé en ce qu'on introduit dans ce vecteur un gène phléo$^r$ de résistance à un antibiotique de la famille des phléomycines, constitué de tout ou partie de Tn5 ou de pUB110 ou d'un gène phléo$^r$ provenant d'un chromosome d'un organisme produisant un antibiotique de la famille des phléomycines, ainsi que les éléments éventuellement nécessaires pour assurer son expression dans les cellules en cause, et en ce que, après transformation ou transfection, on soumet les cellules obtenues à l'action d'un antibiotique de la famille des phléomycines et en ce que l'on sélectionne les cellules résistantes.

12. Procédé de sélection d'une cellule procaryote transformée ou transfectée par un vecteur de clonage et/ou d'expression

ayant intégré un segment d'ADN, caractérisé en ce qu'on introduit ledit segment d'ADN dans le gène phléo$^r$ de résistance aux antibiotiques de la famille des phléomycines, constitué de tout ou partie de Tn5 ou de pUB110 ou d'un gène phléo$^r$ provenant d'un chromosome d'un organisme produisant un antibiotique de la famille des phléomycines, et porté par ledit vecteur et on soumet les cellules transformées ou transfectées à l'action d'un antibiotique de la famille des phléomycines et on sélectionne les cellules sensibles.

## Patentansprüche

1. Verwendung von Antibiotika der Familie der Phleomycine als Selektionsmittel:

— für Eukaryoten-Zellen, die künstlich durch Einführen eines Phleo$^r$-Gens, das resistent gegen ein Antibiotikum der Familie der Phleomycine ist, modifiziert wurden oder

— für Prokaryoten-Zellen, die künstlich modifiziert wurden durch Einführen eines Phleo$^r$-Gens, das gänzlich oder teilweise aus Tn5 oder pUB110 besteht, oder eines Phleo$^r$-Gens, das von einem Chromosom eines Organismus stammt, der ein Antibiotikum der Familie der Phleomycine produziert.

2. Verwendung nach Anspruch 1, dadurch gekennzeichnet, daß die in Frage stehenden Zellen transformiert oder transfektiert wurden mit einem Klonierungs- oder Expressionsvektor, der das Phleo$^r$-Gen trägt, das sich in den genannten Zellen exprimiert.

3. Verwendung nach Anspruch 1, dadurch gekennzeichnet, daß das Phleo$^r$-Gen in ein Chromosom der genannten Zellen integriert wurde.

4. Verwendung nach einem der Ansprüche 1 bis 3, dadurch gekennzeichnet, daß das Phleo$^r$-Gen gänzlich oder teilweise aus Tn5 oder pUB110 besteht.

5. Verwendung nach einem der Ansprüche 1 bis 3, dadurch gekennzeichnet, daß das Phleo$^r$-Gen von einem Chromosom eines Organismus stammt, der ein Antibiotikum der Familie der Phleomycine produziert.

6. Verwendung gemäß einem der Ansprüche 1 bis 5, dadurch gekennzeichnet, daß die Zellen Prokaryoten-Zellen sind.

7. Verwendung nach einem der Ansprüche 1 bis 5, dadurch gekennzeichnet, daß die Zellen Eukaryoten-Zellen sind.

8. Verwendung nach einem der Ansprüche 1 bis 7, dadurch gekennzeichnet, daß man das Selektionsmittel in einer Konzentration von 0,1 µg/ml bis 100 µg/ml in dem Kulturmedium der künstlich modifizierten Zellen verwendet.

9. Verfahren zur Selektion einer mit einem Klonierungs- und/oder Expressionsvektor transformierten oder transfektierten Eukaryoten-Zelle, dadurch gekennzeichnet, daß man in den Vektor ein Phleo$^r$-Gen, das resistent gegen ein Antibiotikum der Familie der Phleomycine ist, sowie die gegebenenfalls zur Sicherung seiner Expression in den infrage kommenden Zellen notwendigen Elemente einführt, und daß man nach Transformation oder Transvektion die erhaltenen Zellen der Einwirkung eines Antibiotikums der Familie der Phleomycine unterwirft und daß man die resistenten Zellen selektiert.

10. Verfahren zur Selektion einer mit einem Klonierungs- und/oder Expressionsvektor transformierten oder transfektierten Eukaryoten-Zelle, die ein DNA-Segment integriert hat, dadurch gekennzeichnet, daß man das DNA-Segment in das Phleo$^r$-Gen, das gegen Antibiotika der Familie der Phleomycine resistent ist, das von dem Vektor getragen wird, einführt, und daß man die transformierten oder transfektierten Zellen der Einwirkung eines Antibiotikums aus der Familie der Phleomycine unterwirft und die sensiblen Zellen selektiert.

11. Verfahren zur Selektion einer mit einem Klonierungs- und/oder Expressionsvektor transformierten oder transfektierten Prokayroten-Zelle, dadurch gekennzeichnet, daß man in den Vektor ein Phleo$^r$-Gen, das resistent gegen ein Antibiotikum aus der Familie der Phleomycine ist, das gänzlich oder teilweise aus Tn5 oder pUB110 besteht, oder ein Phleo$^r$-Gen, das von einem Chromosom eines Organismus stammt, der ein Antibiotikum der Familie der Phleomycine produziert, sowie die gegebenenfalls zur Sicherung seiner Expression in den infrage kommenden Zellen notwendigen Elemente einführt, und daß man nach Transformation oder Transfektion die erhaltenen Zellen der Einwirkung eines Antibiotikums der Familie der Phleomycine unterwirft und daß man die resistenten Zellen selektiert.

12. Verfahren zur Selektion einer mit einem Klonierungs- und/oder Expressionsvektor transformierten oder transfektierten Prokaryoten-Zelle, die ein DNA-Segment integriert hat, dadurch gekennzeichnet, daß man das DNA-Segment in das Phleo'-Gen, das gegen Antibiotika der Familie der Phleomycine resistent ist, das gänzlich oder teilweise aus Tn5 oder pUB110 besteht, oder ein Phleo'-Gen, das von einem Chromosom eines Organismus stammt, der ein Antibiotikum der Familie der Phleomycine produziert, und von dem Vektor getragen wird, einführt, und die tranformierten oder transfektierten Zellen der Einwirkung eines Antibiotikums der Familie der Phleomycine unterwirft und die sensiblen Zellen selektiert.

## Claims

1. Application of the antibiotics of the family of phleomycines as selection agent:
— of eukariotic cells having been modified artificially by incorporation of a phleo' gene of resistance to an antibiotic of the phleomycine family or
— of cells prokariotic cells having been modified artificially by incorporation of a phleo' gene constituted by any or part of Tn5 or pUB110 or a phleo' gene coming from a chromosome of an organism producing an antibiotic of the family of the phleomycine.

2. Application according to claim 1, characterized in that the cells concerned have been transformed or transfected by a cloning or expression vector carrying a phleo' gene which is expressed in said cells.

3. Application according to claim 1, characterized in that the phleo' gene has been integrated into a chromosome of said cells.

4. Application according to one of claims 1 to 3, characterized in that the phleo' gene is constituted by any or part of Tn5 or pUB110.

5. Application according to one of claims 1 to 3, characterized in that the phleo' gene comes from a chromosome of an organism producing an antibiotic of the family of phleomycines.

6. Application according to one of claims 1 to 5, characterized in that the cells are prokaryotic cells.

7. Application according to one of claims 1 to 5, characterized in that the cells are eukariotic cells.

8. Application according to one of claims 1 to 7, characterized in that the selection agent is used at a concentration comprised between 0,1 µg/ml and 100 µg/ml in the culture medium of artificially modified cells.

9. Method of selection of a eukaryotic cell transformed or transfected by a cloning and/or expression vector of a determined protein, characterized in that there is introduced into this vector a phleo' gene of resistance to an antibiotic of the family of phleomycines as well as the elements possibly necessary to ensure its expression in the cells concerned, and in that, after transformation or transfection, the cells obtained are subjected to the action of an antibiotic of the family of phleomycines and the resistance cells are selected.

10. Method of selection of eukariotic cells transformed or transfected by a cloning and/or expression vector of a determined protein having integrated a DNA segment, characterized in that said DNA segment is introduced into the phleo' gene of resistance to the antibiotics of the family of phleomycines borne by said vector and the transformed or transfected cells are subjected to the action of an antibiotic of the family of phleomycines and the sensitive cells are selected.

11. Method of selection of a prokariotic cell transformed or transfected by a cloning and/or expression vector characterized in that there is introduced into this vector a phleo' gene of resistance to an antibiotic to the family of phleomycines constituted by any or part of Tn5 of the phleomycines family as the elements possibly necessary to ensure its expression in the cells concerned, and in that, after transformation or transfection, the cells obtained are subjected to the action of an antibiotic of the family of phleomycines and the resistance cells are selected.

12. Method of selection of a prokariotic cell transformed or transfected by a cloning and/or expression vector having integrated a DNA segment in the phleo' gene of resistance to an antibiotic of the family of phleomycines constituted by any or part of Tn5 or pUB110 or a gene coming from an organism producing an antibiotic of the phleomycines family borne by said vector and the transformed or transfected cells are subjected to the action of an antibiotic of the family of phleomycines and the sensitive cells are selected.

EP 0 193 555 B1

FIG_1

pUT3

HincII
ScaI
PvuI
PstI
XmnI
EcoRI
ClaI
HindIII
PvuII
BglII
BclI
PstI
BalI
MstI
PvuII
SphI
Amp^r
APHK3')II
Bleo^r
5373bp
SmaI (AvaI)
PvuII
PstI
SalI (HindII)
TaqI
HgiEII
Ori
XmaIII
NruI
NdeI
HgiEII
SnaI AccI
Tth111I
PvuII XmnI
BalI AvaI

FIG-3

pUT 100

XbaI
AvaI
BstNI
BamHI
BglI
AvaI
HindII
XhoI (AvaI)
SalI (HindII)
PstI
PvuII
SmaI (AvaI)
EcoRI
APHK3')II
1505 bp
SphI
PvuII
MstI
BalI
PstI
BclI
BglII
HhaI
BstNI
HpaII
HincII

1

```
      10        20        30        40        50        60
TTCGAAATGACCGACCAAGCGACGCCCAACCTGCCATCACGAGATTTCGATTCCACCGCC
AAGCTTTACTGGCTGGTTCGCTGCGGGTTGGACGGTAGTGCTCTAAAGCTAAGGTGGCGG


      70        80        90        100       110       120
GCCTTCTATGAAAGGTTGGGCTTCGGAATCGTTTTCCGGGACGCCGGCTGGATGATCCTC
CGGAAGATACTTTCCAACCCGAAGCCTTAGCAAAAGGCCCTGCGGCCGACCTACTAGGAG


      130       140       150       160       170       180
CAGCGCGGGGATCTCATGCTGGAGTTCTTCGCCCACCCCGGGCTCGATCCCCTCGCGAGT
GTCGCGCCCCTAGAGTACGACCTCAAGAAGCGGGTGGGGCCCGAGCTAGGGGAGCGCTCA


      190       200       210       220       230       240
TGGTTCAGCTGCTGCCTGAGGCTGGACGACCTCGCGGAGTTCTACCGGCAGTGCAAATCC
ACCAAGTCGACGACGGACTCCGACCTGCTGGAGCGCCTCAAGATGGCCGTCACGTTTAGG


      250       260       270       280       290       300
GTCGGCATCCAGGAAACCAGCAGCGGCTATCCGCGCATCCATGCCCCCGAACTGCAGGAG
CAGCCGTAGGTCCTTTGGTCGTCGCCGATAGGCGCGTAGGTACGGGGGCTTGACGTCCTC


      310       320       330       340       350       360
TGGGGAGGCACGATGGCCGCTTTGGTCGACCCGGACGGGACGCTCCTGCGCCTGATACAG
ACCCCTCCGTGCTACCGGCGAAACCAGCTGGGCCTGCCCTGCGAGGACGCGGACTATGTC


      370       380       390       400       410       420
AACGAATTGCTTGCAGGCTACTCATGAGTGTGTCTTCCCGTTTTCCGCCTGAGGTCACTG
TTGCTTAACGAACGTCCGATGAGTACTCACACAGAAGGGCAAAAGGCGGACTCCAGTGAC


      430       440       450       460       470       480
CGTGGATGGAGCGCTGGCGCCTGCTGCGCGACGGCGAGCTGCTCACCACCCACTCGAGCT
GCACCTACCTCGCGACCGCGGACGACGCGCTGCCGCTCGACGAGTGGTGGGTGAGCTCGA
```

# FIG. 2

# FIG - 4

pUT 201

# FIG - 5

pUT 6

EP 0 193 555 B1

FIG-6    pUT 66

FIG-7    pUT 8

4

FIG -8

pUT 301

FIG -9

pUT 400

5

EP 0 193 555 B1

pKC

1195 HindIII
1515 BglII
2034 SphI
2361 AsuII
2684 SalI
2796 HaeI
2832 XhoI
BamHI

ATG 1551
TGA 2343
ATG 2366
TGA 2744

APH (3')II

Bléo$^r$

Sm$^r$

HindIII BglII SalI — pUT 2

HindIII BglII XhoI — pUT 3

HindIII BglII XhoI/SalI — pUT 3

HindIII BglII ← partie délétée → BglII XhoI/SalI — pUT 37

HindIII BglII XhoI BamHI — pUT 6

EcoRI AsuII XhoI BamHI — pUT 8

FIG - 10